# EUROPEAN PATENT SPECIFICATION

(11) **EP 0 562 638 B1**
(45) Date of publication and mention of the grant of the patent: **14.10.1998**
(21) Application number: 93105070.2
(22) Date of filing: 27.03.1993
(51) Int. Cl.: A61K 7/06, A61K 7/08, A61K 7/50

(54) **Stable conditioning shampoo containing cationic and suspended water-insoluble conditioning agents**
Kationische und suspendierte wasserunlösliche Konditionierungsmittel enthaltendes stabiles Haarpflegeshampoo
Shampooing de conditionnement stable contenant des agents de conditionnement cationiques et suspendus insolubles dans l'eau

(30) Priority: 27.03.1992 US 858712
(43) Date of publication of application: 29.09.1993
(73) Proprietor: HELENE CURTIS, INC., Chicago, Illinois 60610-4713 (US)
(72) Inventor: Rizvi, Riaz, Glendale Heights, Illinois 60139 (US); Fahrenwald, William, Villa Park, Illinois 60181 (US); Patel, Chaitanya, Glen Ellyn, Illinois 60137 (US)
(74) Representative: Griffiths, Helen Sarah

(56) References cited:
- EP-A- 0 468 721
- WO-A-92/10162
- GB-A- 2 177 108

## Description

### FIELD OF THE INVENTION

The present invention relates to a conditioning shampoo composition, to its method of preparation and to a method of shampooing hair that cleanses the hair and imparts improved wet stage and improved dry stage conditioning properties to hair in a single application of the composition. More particularly, the present invention is directed to a conditioning shampoo composition including: (a) an anionic cleansing surfactant, like an alkyl ether sulfate, such as sodium lauryl ether sulfate; (b) a combination of a water-soluble and a water-insoluble conditioning agent including: (i) a water-soluble cationic conditioning agent, such as a cationic guar gum, and (ii) a water-insoluble liquid conditioning agent having a viscosity at 25°C less than about 20 centipoises, a specific gravity less than 1.0, preferably less than about 0.95, and a refractive index of at least about 1.4 at 25°C, such as a fatty ester, e.g., myristyl propionate; (c) a suspending agent for the water-insoluble conditioning agent, and (d) a suitable carrier, such as water. Surprisingly, an aqueous conditioning shampoo composition of the present invention including cationic and anionic components, and the combination of a water-soluble cationic conditioning compound and a water-insoluble lubricating conditioning compound together with a suspending agent for the water-insoluble conditioning compound, effectively resists phase separation and does not exhibit an interaction between the cationic components and the anionic components in the composition. Therefore, the anionic component, the water-soluble cationic component and the water-insoluble lubricating conditioning component are available to effectively cleanse the hair and to impart wet stage and dry stage conditioning properties to the hair.

### BACKGROUND OF THE INVENTION AND PRIOR ART

Soiled human hair is shampooed to remove sebum, that is naturally secreted by the head, as well as soil and other atmospheric contaminants that accumulate on the hair. Sebum, in particular, accumulates on the hair in a relatively short period of time leaving the hair with a greasy, dirty feel and poor manageability. The most effective shampoos, for cleansing the hair to remove the atmospheric contaminants and sebum, are those that contain high lather synthetic anionic detergents, such as the long chain alkyl sulfates and the partially ethoxylated long chain alkyl sulfates. These synthetic anionic detergents are very effective for cleansing the hair but, after rinsing with water, leave the hair with a dried touch, usually called "creak", and result in hair, when wet, that is in an extremely tangled and unmanageable after-shampoo condition.

Thoroughly cleansed hair is extremely difficult to comb, in either the wet or dry state because the individual hair fibers tend to snarl, kink, and interlock with each other. Particularly prior to complete drying of thoroughly cleansed hair, in this after-shampoo stage, the hair is very difficult to comb or brush. Even after complete drying, the thoroughly cleansed hair remains difficult to comb or brush and does not set well. Thoroughly clean, dried hair also has undesirable electrostatic properties in a low humidity atmosphere that causes the hair to "fly away", thereby further reducing the combing or brushing property of the hair. Generally, these above-outlined problems that result from synthetic detergent cleansing of the hair, particularly the high-lather synthetic anionic detergents, have been alleviated either by the after-shampoo treatment of the hair with hair conditioners, for example in the form of a hair rinse, or by including hair conditioners directly within the shampoo composition.

After-shampoo hair conditioning compositions are easily formulated but are inconvenient to use because of the necessity of applying the conditioner to the hair in a separate stage, after shampooing. The preparation of a conditioning shampoo has been more difficult because of inherent incompatibility problems between anionic surfactants and the fatty cationic compounds that are good conditioning agents. Contact between an anionic surfactant and a cationic surfactant or cationic polymer produces a precipitate that forms immediately or causes an interaction between the anionic and cationic compounds that significantly reduces their respective cleaning and conditioning properties. This reduction in cleansing and conditioning effectiveness is observed even in compositions wherein the anionic and cationic compounds do not precipitate from the composition but remain in solution or suspension. The incompatibility between an anionic surfactant and a cationic conditioning compound is well recognized by those skilled in the art. For example, Safarin in *Cosmetics*, Interscience Publishers, Inc., New York, p. 538 (1957), states that anionic and cationic compounds cannot be used in combination because they react to form insoluble salts.

Further, while cationic polymers improve wet hair combing, they have a tendency to induce static charge and fly-away resulting in poor dry hair combing and poor dry hair lubrication. Previous attempts to add oily emollient-type conditioning compounds capable of improving dry comb and dry lubrication, such as lanolin, fatty alcohols, or mineral oil together with the cationic polymer has not been effective since the oily conditioning compound does not deposit sufficiently onto the hair, but is washed away with the shampoo composition.

A partial solution to the incompatibility problem in the formulation of conditioning shampoos is exemplified by the following patents that disclose compositions that contain surfactants that are not anionic, e.g., non-ionics, amphoterics and zwitterionics together with cationic conditioning compounds: U.S. Patent No. 3,990,991 to Gerstein; U.S. Patent No. 2,950,255 to Goff; U.S. Patent No. 3,816,616 to Anguillo, et al.; U.S. Patent No. 4,061,602 to Oberstar, et al.; U.S. Patent No. 4,273,760 to Koehler, et al.; U.S. Patent No. 4,247,538 to Barker; U.S. Patent No. 4,479,893 to Hirota, et al. and U.S. Patent No. 3,822,312 to Sato. However, the nonionic, amphoteric and zwitterionic surfactants are inferior cleansing surfactants compared to the anionic surfactants.

Another problem inherent in formulating a conditioning shampoo is an instability problem that results when water-insoluble conditioning agents are also included in the conditioning shampoo composition, such as the non-volatile silicones that are well recognized in the art as providing a degree of softness to the hair.

Silicones in shampoo compositions have been disclosed in a number of different patents: U.S. Patent No. 2,826,551 to Green; U.S. Patent No. 3,964,500 to Drakoff; U.S. Patent No. 4,364,837 to Pader; British Patent No. 849,433 to Woolston; U.S. Patent No. 4,741,855 to Grote, et al.; U.S. Patent Nos. 4,788,006 and 4,902,499 to Bolich, Jr., et al. and U.S. Patent No. 4,704,272 to Oh, et al.

EP-A-0 468 721 teaches a conditioning shampoo composition which comprises a surfactant, an aqueous emulsion of a solution of highly viscous silicone in a volatile solvent and a cationic guar gum. This known composition does not require an emollient ester, but a viscous silicone resin. It teaches that the silicon resin should have a molecular weight between 500 and 10.000. The present application teaches the optional use of silicon gums having 2.000 to 15.000 silicon atoms, this would lead to a minimum molecular weight greater than 100.000 well above the range of said EP-A-0 468 721.

A variety of materials have been proposed for inclusion in silicone-containing conditioning shampoos for purposes of thickening and stabilization such as guar gum, xanthan gum, long chain acyl derivatives, long chain amide oxides, and long chain alkanolamides as disclosed in U.S. Patent Nos. 5,034,218; 4,788,006; 4,704,272; and 4,741,855.

### SUMMARY OF THE INVENTION

In accordance with the principles of the present invention, it has been found, surprisingly that an aqueous hair conditioning shampoo comprising an emulsion of water; 5 % to 65 % by weight of an anionic cleansing surfactant; from 0.1 % to 10 % by weight of a water-soluble or water-miscible cationic conditioning compound comprising a cationic guar gum; from 0.01 % to 15 % by weight of a suspended water-insoluble conditioning compound, namely a water-insoluble emollient ester having a viscosity at 25 °C less than 20 mPas. (centipoises), a specific gravity less than 1.0, a refractive index at 25 °C of at least 1.4, and capable of lubricating hair; and from 0.1% to 15 % by weight of a suspending agent for the water-insoluble conditioning compound has extended product stability, excellent cleansing properties and provides unexptected overall conditioning to human hair, particularly superior wet and dry combing properties.

Surprisingly and unexpectedly, compositions of the present invention are stable and do not exhibit the inherent anionic surfactant/cationic conditioning agent incompatibility. Particularly surprising is that the composition effectively deposits the water-insoluble conditioning agent onto the hair to substantially improve dry comb and dry lubrication properties that the water-soluble cationic conditioning agent does not substantially improve. It was further surprisingly and unexpectedly found that hair treated with the composition of the present invention is thoroughly cleansed and exhibits improved physical and cosmetic properties, such as gloss, thickness, manageability, softness and body. Further, hair treated with the compositions of the present invention does not experience build-up on the hair shaft, over time, of conditioning agents, as is common with many conditioning shampoo compositions.

Therefore, an aspect of the present invention is to provide a hair-treating composition that cleanses the hair and imparts improved physical properties and cosmetic properties to the hair in a single application.

Another aspect of the present invention is to provide a physically stable conditioning shampoo containing an anionic surfactant, a cationic conditioning compound, and a water-insoluble liquid conditioning compound having a viscosity at 25°C less than 20 mPa·s (centipoises), and a specific gravity less than 1.0, preferably less than 0.95; and a suspending agent for the water-insoluble conditioning compound, so that the water-insoluble conditioning compound is suspended in the composition, but sufficiently deposits onto the hair during shampooing without being washed away to substantially improve dry combing and lubrication of dry hair.

Another aspect of the present invention is to provide a new and improved conditioning shampoo containing a strong anionic detergent, such as a long chain alkyl sulfate, long chain alkyl ether sulfate, and/or long chain sulfonate, that is compatible with a water-soluble cationic conditioning compound and a water-insoluble conditioning compound capable of improving the dry combing and dry lubrication properties of the hair, that is stable, but capable of substantial deposition of the water-insoluble conditioning compound onto the hair.

Still another aspect of the present invention is to provide a new and improved conditioning shampoo including 5% to 65% by weight of an anionic surfactant, preferably 5% to 30% by weight of the composition; 0.1% to 10% by weight, preferably 0.1% to 3% by weight of a water-soluble cationic conditioning agent, particularly a cationic polymeric conditioning agent, such as quaternized guar gum; 0.01% to 15% by weight, more preferably 0.01% to 5.0% by weight of the composition, of a water-insoluble liquid conditioning agent having a specific gravity below 1.0, preferably below 0.95, having a viscosity at 25°C less than 20 mPa·s (Centiposes), and having a refractive index of at least 1.4 at 25°C, such as myristyl propionate; and a solid suspending agent for the water-insoluble conditioning agent, such as ethylene glycol monostearate, in an amount of 0.1% to 10% preferably 0.1% to 5% by weight.

A further aspect of the present invention is to provide a new and improved method of shampooing and conditioning hair which comprises applying to said hair the aqueous hair conditioning shampoo of the present invention and thereafter rinsing the hair with water leaving a portion of the cationic and water-insoluble conditioning compounds in contact with hair.

The above and other aspects and advantages of the present invention will become apparent from the following detailed description of the preferred embodiments.

### DETAILED DESCRIPTION OF THE PREFERRED EMBODIMENTS

The aqueous conditioning shampoo composition of the present invention generally includes an anionic surfactant in an amount of 5% to 65% by weight, preferably 5% to 30% by weight, and more preferably 7% to 20% by weight of the composition; a water-soluble cationic conditioning agent, preferably a cationic polymeric conditioning agent, in an amount of 0.1% to 10 % by weight of the composition; a water-insoluble liquid conditioning agent having a specific gravity less than 1.0, preferably less than 0.95, having a viscosity at 25°C less than 20 mPa·s (centipoises), and having a refractive index at 25°C of at least 1.4, and generally in the range of 1.4 to 1.5, in an amount of 0.01% to 15% by weight, preferably 0.01% to 5.0% by weight of the composition; and a solid suspending agent for the water-insoluble conditioning agent, such as ethylene glycol monostearate, in an amount of 0.1% to 10%, preferably 0.1% to 5% by weight of the composition.

The conditioning shampoo of the present invention provides the hair with improved physical and cosmetic conditioning properties, such as gloss, thickness, softness, and manageability, including excellent wet and dry combing properties and body. As will be demonstrated more fully hereinafter, it is surprising and unexpected that the composition of the present invention, including an anionic cleansing compound, a water-soluble or water-dispersible cationic conditioning compound, and a water-insoluble dry hair-lubricating conditioning compound remains stable, while achieving excellent cleansing; and simutaneously provides both wet and dry conditioning properties due to substantive deposition of both the cationic and water-insoluble lubricating conditioning compounds onto the hair.

The anionic cleansing surfactant used in the composition and method of the present invention includes any of the anionic surfactants known or previously used in the art of hair shampoos. An anionic cleansing surfactant should be included in the composition of the present invention to effectively cleanse the hair and generates a high, stable foam level that consumers equate with cleaning efficiency. Nonionic and amphoteric surfactants are not as effective in cleansing the hair and do not provide the high foam level desired by consumers. However, optionally, nonionic, amphoteric and/or zwitterionic surfactants can be included in the compositions of the present invention in addition to one or more anionic surfactants, to help stabilize foam, to provide a suitable viscosity, or to give other functional or esthetic properties to the composition.

Usually, the anionic cleansing surfactant includes a hydrophobic moiety, such as a carbon chain including from about 8 carbon atoms to about 30 carbon atoms, and particularly from about 12 carbon atoms to about 20 carbon atoms and further includes a hydrophilic moiety, such as a sulfate, sulfonate, carbonate, phosphate or carboxylate. Often, the hydrophobic carbon chain is etherified, such as with ethylene oxide or propylene oxide, to impart a particulate physical property, such as increased water-solubility or reduced surface tension, to the anionic cleansing surfactant.

Suitable anionic cleansing surfactants include, but are not limited to, compounds in the classes known as alkyl sulfates, alkyl ether sulfates, alkyl ether sulfonates, sulfate esters of an alkylphenoxy polyoxyethylene ethanol, alpha-olefin sulfonates, beta-alkyloxy alkane sulfonates, alkyl arylsulfonates, alkyl carbonates, alkyl ether carboxylates, fatty acids, sulfosuccinates, alkyl ether sulfosuccinates, sarcosinates, octoxynol or nonoxynol phosphates, taurates, fatty taurides, sulfated monoglycerides, fatty acid amino polyoxyethylene sulfates and isothienates; or combinations thereof. Many additional anionic cleansing surfactants are described in McCUTCHEON'S DETERGENTS and EMULSIFIERS, 1989 ANNUAL published by McCutcheon's Division MC Publishing Company, herein incorporated by reference.

Usually, the anionic cleansing surfactant is present in the composition as a neutralized salt in the form of a sodium potassium, lithium, ammonium, alkylammonium or hydroxyalkylammonium salt, wherein the alkyl moiety includes from 1 to about 3 carbon atoms. The alkyl sulfates and alkyl ether sulfates are particularly effective classes of anionic cleansing surfactants. Exemplary anionic cleansing surfactants that are useful in the composition and method of the present invention include, but are not limited to, the ammonium, monoethanolamine, diethanolamine, triethanolamine, isopropylamine, sodium, potassium, lithium, or magnesium salts of lauryl sulfate, dodecylbenzenesulfonate, lauryl sulfosuccinate, lauryl ether sulfate, lauryl ether carboxylate, lauryl sarcosinate, cocomethyl tauride, and sulfosuccinate half ester amide; or combinations thereof. An especially useful anionic cleansing surfactant is a mixture of a lauryl sulfate salt and a lauryl ether sulfate salt.

In accordance with preferred embodiment of the present invention, the anionic cleasing surfactant is present in the composition in an amount of 5% to 30% by weight of the composition. If the anionic cleansing surfactant is present in an amount of less than 5% by weight of the composition, then the hair is not sufficiently cleansed upon contact with a composition of the present invention. Furthermore, if the anionic cleasing surfactant is present in an amount greater than 30% by weight of the composition, the anionic cleansing surfactant either may form a complex with the cationic conditioning component of the composition, thereby leading to precipitation of the complex, or may solubilize a portion of the water-insoluble conditioning compound, therefore making the solubilized portion essentially unavailable for deposition onto the hair shaft during shampooing. The anionic cleansing surfactant is included in the conditioning shampoo composition of the present invention in a more preferred amount of 7% to 20% by weight of the composition, and to achieve the full advantage of the present invention, from 9% to 18% by weight of the composition.

In accordance with an important feature of the present invention, the hair conditioning shampoo composition includes a water-soluble or water-miscible, cationic conditioning compound that is substantive to the hair and imparts a lubricating conditioning property to dry hair. Preferably, the water-soluble or water-miscible cationic conditioning agent is a polymer. The cationic conditioning compound preferably is water-soluble and is included in the composition in an amount of 0.1% to 10% by weight, and preferably 0.1% to 5% by weight of the composition. Water-soluble or water-dispersible synthetic or naturally-derived cationic polymers having a quaternized nitrogen atom have been found to be particularly useful in the composition and method of the present invention. Such useful polymers have a weight average molecular weight of at least 100,000, and preferably of at least 200,000, and up to about 1,000,000. A preferred weight average molecular weight is from 250,000 to 750,000.

Synthetic quaternized cationic polymers useful in the composition of the present invention include, but are not limited to, polyquaternium-1, polyquaternium-2, polyquaternium-5, polyquaternium-6, polyquaternium-7, polyquaternium-8, polyquaternium-9, polyquaternium-11, polyquaternium-12, polyquaternium-13, polyquaternium-14, polyquaternium-15, and mixtures thereof, wherein the compound designation is the name adopted for the compound by the Cosmetic, Toiletry and Fragrance Association, and found in the *CTFA Cosmetic Ingredient Dictionary*, published by the Cosmetic, Toiletry and Fragrance Association, Inc., Washington, D.C. (1982), or in the 1985 Supplement, hereinafter *The CTFA Dictionary*.

In particular, the naturally-derived quaternised polymers are especially useful in the composition and method of the present invention. Examples of the quaternized naturally-derived polymers include, but are not limited to, the quaternized cellulose and guar-based compounds, compounds designed in *The CTFA Dictionary* as polyquaternium-4, polyquaternium-10, guar hydroxypropyltrimonium chloride, and mixtures thereof. In addition, the synthetic and the naturally-derived quaternized polymers can be used in combination. An especially useful quaternized polymer is guar hydroxypropyltrimonium chloride, sold commercially under the brandname HI-CARE 1000, by Alcolac, Linthicum, MD. Other commercially-available quaternized guar-based conditioning agents include JAGUAR C-13, JAGUAR C-14-S, JAGUAR C-15 and JAGUAR C-17, from Celanese Plastics and Specialties Co., Louisville, Kentucky. Useful quaternized cellulosic compounds include, but are not limited to CELQUAT H60 and CELQUAT L200, from national Starch and Chemical Corp., Bridgewater, New Jersey and QUATRISOFT LM-200 from Amerchol Corp., Edison, New Jersey. Other cationic polymers include a polymer of dimethyl diallyl ammonium chloride, e.g., MERQUAT-100; a polymeric quaternary salt of acrylamide and dimethyl diallyl ammonium chloride, e.g., MERQUAT-550; or a copolymer of vinyl pyrrolidone/methyl vinylimidazolium chloride, e.g., LUVIQUAT HM552.

Compositions including a nonsubstantive, nonionic polymer, like methocel and hydroxypropylcellulose, as opposed to a quaternized guar gum, do not impart sufficient conditioning properties to treated hair, and also are unstable leading to a relatively fast phase separation. In contrast, the water-soluble cationic conditioning compounds, including the preferred polymeric cationic conditioning compounds included in the present composition are substantive to the hair, and also help stabilize the composition.

In accordance with an important feature of the present invention, it has been found that excellent and unexpected substantivity of a suspended water-insoluble (less than 1% by weight soluble in 25°C water) lubricating conditioning compound is achieved so long as the suspended water-insoluble conditioning agent (a) has a viscosity of less than 20 mPa·s (centipoises) at 25°C, (b) has a specific gravity less than 0.95, and (c) has a refractive index of at least 1.4 at 25°C. Water-insoluble conditioning compounds that meet (a) through (c), when suspended in a composition containing a cationic, water-soluble or water-dispersible conditioning compound, preferably a water-soluble polymeric conditioning compound, and a strong anionic cleansing surfactant, will deposit onto the hair together with the cationic conditioning compound. Surprisingly, the water-insoluble conditioning compound is not washed away with the surfactant(s) and soil when the hair is rinsed during the shampooing process.

The preferred water-insoluble conditioning compounds useful in accordance with the present invention include any ester including at least 12 carbon atoms in the molecule, preferably including a long chain C₁₂-C₂₄ alkyl moiety, and having emollient properties for lubricating hair to improve the dry comb or dry lubrication properties of the hair. For example, other suitable esters include myristyl propionate, isopropyl myristate, archidyl propionate, butyl stearate, butyl myristate, butyl oleate, C₁₂-C₁₅ alcohols benzoate and C₁₈-C₂₀ glycol isostearate.

Other suitable esters include the polycarboxylate esters, such as diisopropyl sebacate, diisopropyl adipate and triisocetyl citrate. The polycarboxylate esters having at least one long chain also are suitable, such as pentaerythritol tetracaprate/caprylate, pentaerythritol tetraisostearate, and propylene glycol di-capric/caprylate.

Other useful water-insoluble conditioning compounds include nonvolatile and/or volatile silicone conditioning compounds, and nonvolatile or volatile hydrocarbon conditioning compounds, so long as the silicone and hydrocarbon compounds have a viscosity at 25°C less than 20mPa·s (centipoises), a specific gravity less than 1.0, preferably less than 0.95, and a refractive index of at least 1.4 at 25°C.

In addition, cyclic, volatile polydimethylsiloxanes, designated in the *CTFA Dictionary* as cyclomethicones, also are useful in the composition and method of the present invention as the water-insoluble conditioning compound. The cyclomethicones are low molecular weigh, water-insoluble cyclic compounds having an average of about 3 to about 6 -[O-Si(Ch₃)₂]- repeating group units per molecule and boil at atmospheric pressure in a range of from about 150°C to about 250°C. The volatile cyclomethicone silicone compound can be, for example, a tetramer, pentamer and/or hexamer of cyclomethicone. Mixtures of these silicone conditioning compounds also are useful. Suitable cyclomethicones are available commercially under the tradenames SILICONE SF-1173 (octamethylcyclotetrasiloxane) and SILICONE SF-1202 (decamethylcyclopentasiloxane) from General Electric, Waterford, New York, and SILICONE 334 FLUID and SILICONE 345 FLUID from Dow Corning Corporation, Midland, Michigan, the tetramer being listed first in each instance.

Another suitable water-insoluble conditioning agent useful in the composition of the present invention is a water-insoluble hydrocarbon conditioner, such as low viscosity mineral oils, C₁₁-C₁₅ isoparaffins, isododecane, and isohexadecane. The nonvolatile hydrocarbons provide many of the sane benefits as the silicone conditioning compounds, and can be included in the composition in conjunction with a silicone conditioning compound.

In another embodiment, the water-insoluble conditioning agent is a hydrocarbon conditioner, such as a hydrocarbon including 10 to 45 carbon atoms, that has sufficient volatility to slowly volatilize from the hair to prevent a residual buildup of hydrocarbon on dry hair. The volatile hydrocarbon provides essentially the same benefits as the volatile silicone, such as lubrication and wet hair conditioning.

The preferred volatile hydrocarbon compound is an aliphatic hydrocarbon including 12 to 24 carbon atoms, and has a boiling point in the range of from 100°C to 300°C. Exemplary volatile hydrocarbons are depicted in general structural formula (I), wherein n ranges from 2 to 5.

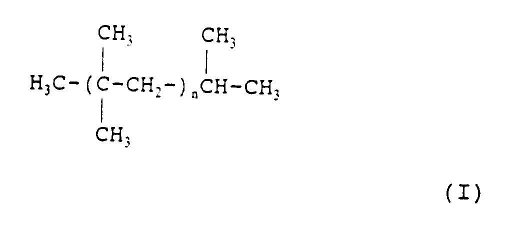

Examples of volatile hydrocarbons useful in the compositions of the present invention are the commercially-available compounds PERMETHYL 99A and PERMETHYL 101A, corresponding to compounds of general structure (I) wherein n is 2 and 3, respectively, from Permethyl Corporation, Frazer, Pennsylvania. A volatile hydrocarbon compound is useful in the conditioning shampoo composition of the present invention either alone, in combination with another volatile or nonvolatile hydrocarbon, or in combination with a volatile or nonvolatile silicone.

The water-insoluble emollient-type conditioning compound(s) included in the conditioning shampoos of the present invention are suspended with 0.10% to 15.0% by weight, preferably 0.10% to 5.0% by weight of the composition, of an emulsifier or suspending agent to stabilize the composition and sometimes to add a pearlescent appearance to the composition. The suspending agent should be a solid at room temperature (25°C) and includes the long chain (C₁₂-C₄₅) alkyl-containing compounds, including long chain alcohols, esters, acids, and their derivatives; synthetic or natural waxes that include long chain alcohols, esters and/or acids, for example SYNCROWAX AW1-C, MONTAN acid wax, containing about 75% by weight behenic acid, and hydrocarbon waxes, such as paraffinic waxes.

Exemplary solid fatty alcohol (C₁₆-C₄₀) suspending agents for the water-insoluble conditioning compound include, but are not limited to, tallow alcohol, cetyl alcohol, stearyl alcohol, cetearyl alcohol, behenyl alcohol, and combinations thereof

In another embodiment, the solid suspending agent for the water-insoluble conditioning compound is a fatty ester having a general formula (II) 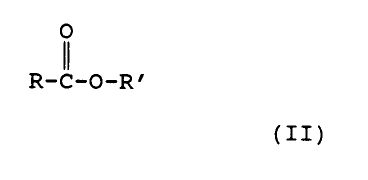 wherein R or R' are straight or branched chains alkyl or substituted alkyl radicals or R' is a glycol residue and wherein one or both R and R' have about 12 to about 24 carbon atoms. The fatty component of the fatty ester can be derived from a fatty acid or a fatty alcohol, or a combination thereof. Exemply fatty esters includes, but are not limited to, cetyl palmitate, myristyl stearate, ethylene glycol monostearate, ethylene glycol distearate and mixtures thereof.

Solid fatty acid suspending agents (solid at 25°C) for the water-insoluble conditioning compound useful in the conditioning shapoos of the present invention include primary or secondary fatty acids having 12 to 40 carbons, inclusive, either as single long-chain lengths (e.g., 20, 22, 24, 26, 28, 30, 32, 34, 36, 38 or 40 carbon atoms in length) or as a mixture of long-chain lengths in any combination. The fatty acids can be straight chain, branched, saturated, and/or unsaturated structures and can be used along or in admixture with each other. The preferred fatty acids are straight-chained, primary acids having about 20 to about 40 carbons, or mixtures of 20, 22, 24, 26, 28, 30, 32, 34, 36, 38 and/or 40 carbons, and combinations thereof. In addition, mixtures of natural or synthetic fatty acids having fatty chain lengths of from 20 to 40 carbons, inclusive, also are useful. Such long chain fatty acids are commercially available, for example as SYNCROWAX AW1-C (C₁₈-C₃₆) and MONTAN ACID WAX, fatty wax acids containing about 75% C₂₂ saturated wax acid and about 25% C₁₈, C₂₀, and C₂₄-C₃₆ acids. The acids can be used as the carboxylic acid, or can be neutralized with any base, to form the salt.

The components of the composition are combined and sheared together in any order. To achieve the best results, at the point that the composition is sheared, the composition should have a viscosity in the range of 2,000 to 20 000 mPa s (centipoises) so that upon vigorous mixing or shearing, the resulting droplets of water-insoluble lubricating conditioning agent have a particle size of 5 µm to 100 µm , and preferably at least 10 µm to 50 µm , and are stable in the composition. As an example of the vigorous mixing, a six-bladed axial flow turbine impeller rotating at a speed of 500 to 800 r.p.m. provides sufficient shearing of the composition to homogeneously dissolve or disperse the cationic polymer and suspend the water-insoluble dry hair lubricating conditioning compound resulting in droplets of water-insoluble conditioning compound within the size range of about 20 microns to about 50 microns, and the sheared water-insoluble conditioning compound droplets are exceptionally stable. Depending upon the quantity of suspending agent used, and the amount of water contained in the composition, 0% to 20% of the water contained in the final composition can be withheld from the composition, together with a proportionate percentage of the anionic surfactant, if necessary, to provide a sufficiently viscous composition to provide sufficient shear to the water-insoluble conditioning compound to achieve a particle size of 5 µm to 100 µm for good substantive deposition of the water-insoluble conditioning compound, e.g. 2,000 to 20,000 mPa·S (centipoises).

The shearing of the water-soluble conditioning compound within a viscous composition provides the best conditioning compound particle size and stability to the composition. If the particle size of the water-insoluble lubricating conditioning compound droplets is less than about 5 µm, the total surface area is too great resulting in the lubricating conditioning compound being rinsed away with the anionic surfactants and, therefore, insufficient substantivity to the hair. If the particle size of the droplets of water-insoluble lubricating conditioning compound is greater than about 100 µm , the droplets of lubricating conditioning compound have a tendency to coalesce and separate from the composition.

If some of the water and surfactant are withheld from the composition until after shearing of the lubricating conditioning compound to maintain the composition sufficiently viscous during dispersion, the remaining water and anionic surfactant(s) then are added, slowly, e.g., 0,4536 to 1,3608 (1 to 3 pounds) preferably 0,4536 to 0,9072, kg (1 to 2 pounds) per minute for a 113,4 kg (250 pounds) shampoo batch, with gentle agitation.

Optionally, the composition of the present invention can include from 0.15% to 10%, and preferably from 1.0% to 5%, by weight of a non-volatile silicone compound having a viscosity above 20 mPa·S (centipoises) at 25°C. The preferred non-volatile silicone compound is a polydimethylsiloxane compound, such as a mixture, in about a 3:1 ratio, of a low molecular weight polydimethysiloxane fluid and a higher molecular weight polydimethylsiloxane gum. The non-volatile polydimethylsiloxane compound is added to the composition of the present invention in an amount sufficient to provide improved combing and improved feel (softness) to the hair after shampooing. As referred to herein, "silicone gums" are those nonfunctional siloxanes having a viscosity of from 5 to 600,000 centistokes at 25°C. The so-called rigid silicones, as described in U.S. Patent No. 4,902,499, herein incorporated by reference, having a viscosity above 600,000 centistokes at 20°C, e.g., 700,000 centistokes plus, and a weight average molecular weight of at least about 500,000 also are useful in accordance with the present invention.

Preferred silicone gums include linear and branched polydimethylsiloxanes, of the following general formula:

(CH₃)₃SiO--[Si(CH₃)₂O]ₙ--Si(CH₃)₃ ,

wherein n is from 2,000 to 15,000, preferably from 2,000 to 7,000. Silicone gums useful in compositions of the present invention are available from a variety of commercial sources, including General Electric Company and Dow Corning.

The following example illustrates a conditioning shampoo made in accordance with the present invention:

| **EXAMPLE** | | |
|---|---|---|
| Item | Ingredient (CTFA Name) | w/w% |
| 1. | Deionized Water | 49.31 |
| 2. | Hydroxypropyl Guar -Hydroxypropyltrimonium Chloride | 0.300 |
| 3. | Citric Acid 50% | 0.040 |
| 4. | Lauryl Betaine | 2.000 |
| 5. | Ammonium Laureth Sulfate (2 moles of ethoxylation) (30% Active) | 10.000 |
| 6. | Ammonium Lauryl Sulfate (30% Active) | 35.000 |
| 7. | Ethylene Glycol Monostearate (suspending agent) | 0.600 |
| 8. | Cocamide MEA (suspending agent) | 1.500 |
| 9. | Behenic Acid (suspending agent) | 0.100 |
| 10. | Myristyl Propionate (water-insoluble conditioning compound) | 0.500 |
| 11. | Fragrance | 0.500 |
| 12. | Methylchloroisothiazolinone/Methylisothiozolinone (preservative) | 0.050 |
| 13. | Dimethicone Copolyol (water-soluble conditioning agent for viscosity adjustment) | 0.100 |
| SPECIFICATIONS pH 5.3-5.8 Viscosity 4,000 - 7,000 mPa s (centipoises) at 25 °C | | |

## Claims

1. An aqueous hair conditioning shampoo composition comprising an emulsion of water; 5 % to 65 % by weight of an anionic cleansing surfactant; from 0.1 % to 10 % by weight of a water-soluble or water-miscible cationic conditioning compound, comprising a cationic guar gum; from 0.01 % to 15 % by weight of a suspended water-insoluble conditioning compound namely, a water-insoluble emollient ester having a viscosity at 25 °C less than 20 mPas. (centipoises), a specific gravity less than 1.0, a refractive index at 25 °C of at least 1.4, and capable of lubricating hair; and from 0.1% to 15 % by weight of a suspending agent for the water-insoluble conditioning compound.

2. The composition as defined in claim 1, wherein the cationic guar gum is present in an amount of 0.1% to 5.0% by weight of the composition.

3. The composition as defined in claims 1 and 2 wherein the cationic, guar gum is selected from the group consisting of a quaternized guar gum, a hydroxypropylated guar gum and a carboxymethylated guar gum.

4. The composition as defined in claim 3, wherein the cationic guar gum is a hydroxyalkyl guar-hydroxyalkyltrimonium salt.

5. The composition as defined in anyone of claims 1 to 4, wherein said emollient ester is selected from the group consisting of isopropyl myristate, myristyl propionate, C₁₂ - C₁₅ alcohol benzoate, and mixtures thereof.

6. The composition of anyone of claims 1 to 4, wherein the emollient ester is selected from the group consisting of polyhydric alcohol esters, polycarboxylate esters, and mixtures thereof.

7. The composition as defined in claim 4, wherein the cationic guar gum is hydroxypropyl guarhydroxypropyl trimonium chloride.

8. The hair conditioning composition as defined in anyone of claims 1 to 7, wherein the suspending agent for the water-insoluble conditioning compound is a solid at room temperature and is selected from the group consisting of (a) primary or secondary fatty acids or fatty alcohols having about 12 to about 40 carbon atoms in long chain radical, (b) a fatty ester having a long chain C₁₂ - C₃₆ radical, and (c) mixtures of any two or more of (a) -(b).

9. A method of shampooing and conditioning hair which comprises applying to said hair the composition of anyone of claims 1 to 8, and thereafter rinsing the hair with water leaving a portion of the cationic and water-insoluble conditioning compounds in contact with the hair.

## Patentansprüche

1. Eine wässerige haarkonditionierende Shampoo-Zusammensetzung, enthaltend eine Emulsion von Wasser; 5 bis 65 Gewichtsprozent eines anionischen Reinigungssurfactants; von 0,1 bis 10 Gewichtsprozent einer wasserlöslichen oder wassermischbaren kationischen Konditionierverbindung, enthaltend ein kationisches Guar-Mehl; von 0,01 bis 15 Gewichtsprozent einer suspendierten wasserunlöslichen Konditionierverbindung, nämlich einem wasserunlöslichen Erweichungsester mit einer Viskosität bei 25°C von kleiner als 20 mPa.s (Centipoise), einem spezifischen Gewicht von kleiner als 1,0, einem Brechungsindex bei 25°C von zumindest 1,4, und fähig zur Einfettung von Haar; und von 0,1 bis 15 Gewichtsprozent eines Suspendiermittels für die wasserunlösliche Konditionierverbindung.

2. Die Zusammensetzung nach Anspruch 1, worin das Kationische Guar-Mehl in einer Menge von 0,1 bis 5,0 Gewichtsprozent der Zusammensetzung vorhanden ist.

3. Die Zusammensetzung nach einem der Ansprüche 1 und 2, worin das kationische Guar-Mehl aus der Gruppe bestehend aus einem quaternisierten Guar-Mehl, einem hydroxypropylierten Guar-Mehl und einem carboxymethylierten Guar-Mehl ausgewählt ist.

4. Die Zusammensetzung nach Anspruch 3, worin das kationische Guar-Mehl ein Hydroxyalkylguar-hydroxyalkyltrimoniumsalz ist.

5. Die Zusammensetzung nach irgendeinem der Ansprüche 1 bis 4, worin der erwähnte Erweichungsester aus der Gruppe bestehend aus Isopropylmyristat, Myristylpropionat, C₁₁₋₁₅-Alkoholbenzoat, und Mischungen derselben, ausgewählt ist.

6. Die Zusammensetzung nach irgendeinem der Ansprüche 1 bis 4, worin der Erweichungsester aus der Gruppe bestehend aus mehrwertigen Alkoholestern, Polycarboxylatestern, und Mischungen derselben, ausgewählt ist.

7. Die Zusammensetzung nach Anspruch 4, worin das kationische Guar-Mehl Hydroxypropylguarhydroxypropyltrimoniumchlorid ist.

8. Haarkonditionierende Zusammensetzung nach irgendeinem der Ansprüche 1 bis 7, worin das Suspendiermittel für die wasserunlösliche Konditionierverbindung bei Raumtemperatur ein Feststoff ist und aus der Gruppe bestehend aus (a) primären oder sekundären Fettsäuren oder Fettalkoholen mit etwa 12 bis etwa 40 Kohlenstoffatomen in langkettigem Rest, (b) einem Fettester mit einem langkettigen C₁₂₋₃₆-Rest, und (c) Mischungen von irgendwelchen zwei oder mehr Resten von (a) bis (b) ausgewählt ist.

9. Ein Verfahren zum Schampunieren und Konditionieren von Haar, welches das Aufbringen der Zusammensetzung von irgendeinem der Ansprüche 1 bis 8 auf das Haar und das anschließende Spülen des Haars mit Wasser umfaßt, lassend einen Teil der kationischen und wasserunlöslichen Konditionierverbindungen in Kontakt mit dem Haar.

## Revendications

1. Une composition aqueuse de shampooing de conditionnement des cheveux comprenant une émulsion d'eau ; de 5 % à 65 % en masse d'un agent tensioactif nettoyant anionique ; de 0,1 % à 10 % en masse d'un composé de conditionnement cationique soluble dans l'eau ou miscible dans l'eau, comprenant une gomme guar cationique ; de 0,01 % à 15 % en masse d'un composé de conditionnement en suspension et insoluble dans l'eau, à savoir un ester émollient insoluble dans l'eau ayant une viscosité à 25°C inférieure à 20 mPa.s (Centipoises), une gravité spécifique inférieure à 1,0, un indice de réfraction d'au moins 1,4 à 25°C, et qui est capable de lubrifier les cheveux ; et de 0,1 % à 15 % en masse d'un agent de mise en suspension pour le composé de conditionnement insoluble dans l'eau.

2. La composition selon la revendication 1, dans laquelle la gomme guar cationique est présente dans une quantité allant de 0,1 % à 5,0 % en masse par rapport à la composition.

3. La composition selon les revendications 1 et 2, dans laquelle la gomme guar cationique est sélectionnée à partir du groupe composé d'une gomme guar quaternaire, d'une gomme guar hydroxypropylée et d'une gomme guar carboxyméthylée.

4. la composition selon la revendication 3, dans laquelle la gomme guar cationique est un sel hydroxyalkyltrimonium de guar hydroxyalkyle.

5. Une composition selon l'une des revendications 1 à 4, dans laquelle ledit ester émollient est sélectionné à partir du groupe composé de l'isopropyl myristate, du myristyl propionate, du benzoate d'alcool en C₁₂ - C₁₅ et de mélanges de ceux-ci.

6. Une composition selon l'une des revendication 1 à 4, dans laquelle l'ester émollient est sélectionné à partir du groupe composé des esters d'alcool polyhydriques, des esters de polycarboxylates et des mélanges de ceux-ci.

7. Une composition selon la revendication 4, dans laquelle la gomme guar cationique est du chlorure d'hydroxypropyl guarhydroxypropyl trimonium.

8. La composition de conditionnement des cheveux selon l'une des revendications 1 à 7, dans laquelle l'agent de mise en suspension pour le composé de conditionnement insoluble dans l'eau est un solide à température ambiance et est sélectionné à partir du groupe composé de (a) des acides gras primaires ou secondaires ou des alcools gras ayant d'environ 12 à environ 40 atomes de carbone dans un radical à chaîne longue, (b) un ester gras ayant un radical à chaîne longue en C₁₂ - C₃₆, et ( c) des mélanges de deux ou de plusieurs éléments (a) - (b).

9. Un procédé de shampouinage et de conditionnement des cheveux comprenant le fait d'appliquer la composition selon l'une des revendications 1 à 8 sur lesdits cheveux, puis de rincer les cheveux avec de l'eau en laissant une partie des composés cationiques et insolubles dans l'eau en contact avec les cheveux.
